# EUROPÄISCHE PATENTANMELDUNG

(11) **EP 0 742 215 A1**
(43) Veröffentlichungstag der Anmeldung: **13.11.1996**
(21) Anmeldenummer: 96106715.4
(22) Anmeldetag: 29.04.1996
(51) Int. Cl.: C07D 307/92, C12N 1/14, A61K 31/34

(54) **Cillianon, Antibiotikum aus Penicillin spe., und chemische Derivate davon, Verfahren zur Herstellung und Verwendung derselben**

(30) Priorität: 05.05.1995 DE 19516521
(71) Anmelder: HOECHST AKTIENGESELLSCHAFT, 65929 Frankfurt am Main (DE)
(72) Erfinder: Vértesy, Laszlo, Dr., 65817 Eppstein (DE); Fehlhaber, Hans-Wolfram, Dr., 24306 Plön (DE); Jordan, Birgit, Dr., 65795 Hattersheim (DE); Wink, Joachim, Dr., 63322 Rödermark (DE); Lauffer, Leander, Dr., 35075 Gladenbach (DE)

(57) **Zusammenfassung**

Die vorliegende Erfindung betrifft ein neues Antibiotikum mit der Bezeichnung Cillianon, das von dem Pilz Penicillium spec. HAG 0259 während der Fermentation gebildet wird, ein Verfahren zu dessen Herstellung, von Cillianon abgeleitete chemische Derivate, die Verwendung von Cillianon und davon abgeleitete Derivate als pharmakologische Wirkstoffe, als Arzneimittel und insbesondere als Interleukin (IL)-4-Antagonisten sowie Penicillium spec. HAG 0259 zur Herstellung der obengenannten Antibiotika.

## Beschreibung

Die vorliegende Erfindung betrifft ein neues Antibiotikum mit der Bezeichnung Cillianon, das von dem Pilz Penicillium spec. HAG 0259 während der Fermentation gebildet wird, ein Verfahren zu dessen Herstellung, von Cillianon abgeleitete chemische Derivate, die Verwendung von Cillianon und davon abgeleitete Derivate als pharmakologische Wirkstoffe, als Arzneimittel und insbesondere als Interleukin (IL)-4-Antagonisten sowie Penicillium spec. HAG 0259 zur Herstellung der obengenannten Antibiotika.

Sekundärmetabolite aus Mikroorganismen werden erfolgreich zur Behandlung von Infektionskrankheiten eingesetzt. Bei Sekundärmetaboliten handelt es sich um niedermolekulare Verbindungen, deren Bildung in "biosynthetischen Einbahnstraßen", die vom Primärmetabolismus abzweigen, erfolgt und deren Funktion für den jeweiiigen Produzenten ungeklärt ist. Bis heute sind ca. 10000 aus Kulturen verschiedener Mikroorganismen (vor allem Pilze und Bakterien der Gattung Streptomyces) isolierte Sekundärmetabolite bekannt.

Haupteinsatzgebiet dieser Sekundärmetabolite ist die Therapie von Infektionskrankheiten. Durch den breiten Einsatz kommt es jedoch häufig zur Resistenzbildung, so daß ein ständiger Bedarf an neuen Antibiotika und Wirkstoffen mit neuen Wirkmechanismen besteht (Neu H.C., Science 257, 1992, S. 1064-1073).

Daneben hat sich das Indikationsgebiet mikrobieller Wirkstoffe auch auf Krankheiten, die nicht zu den Infektionskrankheiten zählen (z.B. Tumortherapie, Immunmodulation) und auf den Pflanzenschutz (Herbizide und Insektizide) ausgeweitet. Auch ist bekannt, daß Sekundärmetabolite mit einer Vielzahl pharmakologisch interessanter Makromoleküle wie Rezeptoren oder Enzyme von Signalketten interagieren und antagonistische bzw. inhibitorische Wirkungen hervorrufen.
Die eingesetzten Wirkstoffe sind allerdings häufig mit Mängel behaftet, gekennzeichnet durch unbefriedigende Wirkhöhen, eine zu hohe Toxizität und/oder unerwünschte Nebenwirkungen.

In der Literatur sind Antibiotika beschrieben, die sich von den erfindungsgemäßen Antibiotika strukturell unterscheiden (T. Yoshioka et al., Bull. Chem. Soc. Jpn, 1982, 55, 3847-3851). J. Casson et al., J. Org. Chem. 1970, 35, 170-186, berichteten von einem Norherqueinon, dessen ¹H-NMR-Daten, UV-Spektrum und massenspektrometrische Verhalten eindeutig den Unterschied zu den erfindungsgemäßen Verbindungen belegen. Das vor kurzem publizierte Rousselianon (Jin-Zhong-Xiao et al., J. Antibiotics 1993, 46, 1570-1574), welches aus Phaeosphaeria rousseliana isoliert wurde, unterscheidet sich von den erfindungsgemäßen Verbindungen z.B. durch das Fehlen des 1,2,2-Trimethyltetrahydrofuranringes, wie aus den NMR-Signalen hervorgeht.

Aufgabe der Erfindung ist es, nach mikrobiellen Naturstoffen mit verbesserten Eigenschaften zu suchen.

Diese Aufgabe wird erfindungsgemäß gelöst, indem man Penicillium spec. HAG 0259 in einem Nährmedium mit Kohlenstoff- und Stickstoffquelle sowie den üblichen anorganischen Salzen und Spurenelementen fermentiert bis sich Cillianon in der Kulturbrühe anhäuft, anschließend Cillianon aus der Kulturbrühe isoliert und gegebenenfalls chemische Derivate von Cillianon herstellt. Die Antibiotika besitzen pharmakologische Aktivität und damit therapeutische Wirksamkeit und können als Interleukin (IL)-4-Antagonisten (Substanzen, die die IL-4-Wirkung unterdrücken) eingesetzt werden.

Die Erfindung betrifft somit
1. Verbindungen der Formel I 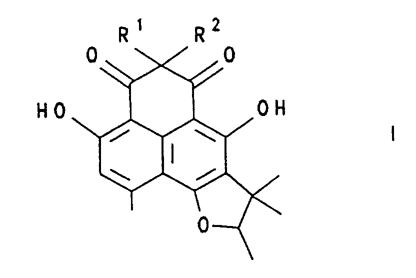 in welcher bedeuten:
   a)
      - R¹ und R²: unabhängig voneinander
      -CₙH₂ₙ-R³, -O-R³, -S-R³, oder NH-R³;
      - R³: Wasserstoff, (C₁-C₆)-Alkanoyl oder einen substituierten oder unsubstituierten (C₁-C₆₎-Alkylrest; und
      - n: 1,2,3,4,5 oder 6; oder
   b) R¹ und R² zusammen mit dem sie tragenden C-Atom einen substituierten oder unsubstituierten alicyclischen Rest mit 3 - 9 C-Atomen bilden,
   sowie deren physiologisch verträglichen Salze.
   In einer bevorzugten Gruppe von Verbindungen der Formel I bedeuten
   - R¹: OH;
   - R²: OH, NH-R³ oder CH₂-R³; und
   - R³: Wasserstoff, Formyl, Acetyl, Carboxymethyl oder Carboxyethyl.

   In einer nochmals bevorzugten Gruppe von Verbindungen der Formel I bedeuten R¹ und R² Hydroxyl.
2. Cillianon, eine Verbindung der Summenformel C₁₉H₁₈O₇, erhältlich aus Penicillium spec. HAG 0259 durch Fermentierung von Penicillium spec. HAG 0259 in einem Kulturmedium, bis sich Cillianon in der Kulturbrühe anhäuft, und anschließender Isolierung und Aufreinigung des Wirkstoffes, sowie dessen pharmakologisch verträglichen Salze.
3. Aus Cillianon abgeleitete chemische Derivate, erhältlich aus Penicillium spec. HAG 0259 durch Fermentierung von Penicillium spec. HAG 0259 in einem Kulturmedium, bis sich Cillianon in der Kulturbrühe anhäuft, anschließender Isolierung und Aufreinigung des Wirkstoffes und Überführung in chemische Derivate, sowie deren pharmakologisch verträglichen Salze.
4. Ein Verfahren zur Herstellung der unter 1. 2. oder 3. definierten Verbindungen, dadurch gekennzeichnet, daß man Penicillium spec. HAG 0259 oder dessen Mutanten und/oder Varianten in einem Kulturmedium fermentiert, bis sich das Cillianon in der Kulturbrühe anhäuft, dieses aus der Kulturbrühe isoliert, und gegebenenfalls in ein chemisches Derivat überführt.
5. Eine Verwendung von Cillianon oder eines daraus abgeleiteten chemischen Derivats als pharmakologisch wirksamen Stoff, insbesondere als IL-4-Antagonist.
6. Penicillium spec. HAG 0259 (DSM 9791)

Im folgenden wird die Erfindung detailliert beschrieben, insbesondere in Ihren bevorzugten Ausführungsformen. Ferner wird die Erfindung durch den Inhalt der Patentansprüche bestimmt.

Definition von Begriffen:

Die erfindungsgemäße Verbindung der Summenformel C₁₉H₁₈O₇ wird Cillianon genannt.
Als Kulturbrühe wird das Nährmedium, welches das darin gewachsene Pilzmycel enthält, bezeichnet.
Alle Prozentangaben beziehen sich, wenn nicht anders angegeben, auf das Gewicht. Mischungsverhältnisse bei Flüssigkeiten beziehen sich auf das Volumen, wenn keine andere Angaben gemacht werden.
Das erfindungsgemäße Verfahren kann für die Fermentation im Labormaßstab (Milliliter- bis Literbereich) und für den industriellen Maßstab (Kubikmetermaßstab) eingesetzt werden.

Penicillium spec. HAG 0259 wurde aus einer Erdprobe isoliert. Für die Aufreinigung aus der Erde wurde diese unter Herstellung einer Verdünnungsreihe mit einer physiologischen NaCl-Lösung (0,9%) aufgeschwemmt. Die verschiedenen Verdünnungen (10⁰-10⁶) wurden anschließend auf Nährböden für Penicillien ausplattiert. Nach einer Bebrütung der Kulturen bei 30° C für die Dauer von 2 bis 14 Tagen entstehen Penicillienkolonien, die vereinzelt und durch mehrere aufeinanderfolgende Aufreinigungsschritte isoliert wurden.
Die Bestimmung der Gattung wird anhand von morphologischen und taxonomischen Kriterien nach dem Fachmann bekannten Methoden vorgenommen. Charakteristisch für die Gattung Penicillium sind deren Conidiophoren.

Aufgrund von aufeinanderfolgenden Isolierungs- und Aufreinigungsschritten kann von von Penicillium spec. HAG 0259 eine Kolonie isolieren, die den erfindungsgemäßen Wirkstoff Cillianon sehr effizient produziert.

Eine stark produzierende Kolonie von Penicillium spec. HAG 0259 wird vermehrt. Ein Isolat wurde bei der Deutschen Sammlung von Mikroorganismen und Zellkulturen GmbH, Mascheroder Weg 1B, 38124 Braunschweig, Deutschland, nach den Regeln des Budapester Vertrages am 16.3.95 unter der folgenden Nummer hinterlegt: Penicillium spec. DSM 9791

Penicillium spec. DSM 9791 besitzt weißes Luftmycel und grüne Conidien. Er bildet die für Penicillien charakteristischen Conidiophoren und zeigt ein watteartiges Wachstum. In einer Nährlösung, die eine Kohlenstoffquelle und eine Stickstoffquelle sowie die üblichen anorganischen Salze enthält, produziert Penicillium spec. DSM 9791 die Verbindung der Summenformel C₁₉H₁₈O₇.

Anstelle des Stammes DSM 9791 können auch dessen Mutanten und Varianten eingesetzt werden, soweit sie die erfindungsgemäße Verbindung synthetisieren. Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) erzeugt werden.

Das Screening nach Mutanten und Varianten, die die erfindungsgemäßen Cillianone produzieren, kann durch Bestimmung der biologischen Aktivität der in der Kulturbrühe angehäuften Wirkstoffe, beispielsweise durch Austesten der IL-4-inhibierenden Wirkung nach dem unten beschriebenen Verfahren erfolgen.

Die im folgenden beschriebenen Fermentationsbedingungen gelten für Penicillium spec. HAG 0259 sowie Mutanten und Varianten von diesen.

Als bevorzugte Kohlenstoffquelle für die aerobe Fermentation eignen sich assimilierbare Kohlenhydrate und Zuckeralkohole, wie Glukose, Laktose oder D-Mannit sowie kohlenhydrathaltige Naturprodukte, wie z.B. Malzextrakt.

Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate.

An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung von Cillianon verläuft besonders gut in einer Nährlösung, die etwa 0,1 bis 3 % bevorzugt 0,2 bis 1 % Caseinpepton; 0,1 bis 3 % bevorzugt 0,2 bis 1 % Fleischpepton; 0,1 bis 5 % bevorzugt 0,5 bis 2 % Glucose, sowie 0,1 bis 7 % bevorzugt 1 bis 2 % Maltose jeweils bezogen auf das Gewicht der gesamten Nährlösung enthält.

Die Kultivierung erfolgt aerob, also beispielsweise submers unter Schütteln oder Rühren in Schüttelkolben oder Fermentern, gegebenenfalls unter Einführen von Luft oder Sauerstoff.
Die Fermentation kann beispielsweise in Steilbrustflaschen oder Rundkolben verschiedener Volumina, in Glasfermentern oder V₂A-Stahltanks durchgeführt werden.
Sie kann in einem Temperaturbereich von etwa 15 bis 30°C, vorzugsweise bei etwa 20 bis 30°C, insbesondere bei 23 bis 28°C durchgeführt werden. Der pH-Wert sollte zwischen 1 und 7 liegen, vorteilhaft zwischen 2,5 und 4,5. Man kultiviert den Mikroorganismus unter diesen Bedingungen im allgemeinen über einen Zeitraum von 24 bis 300 Stunden, bevorzugt 36 bis 140 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man z.B. indem man ein versportes Mycel in eine Nährlösung überimpft und etwa 36 bis 120 Stunden, bevorzugt 48 bis 72 Stunden, wachsen läßt. Das versporte Mycel kann beispielsweise erhalten werden, indem man den Stamm etwa 3 bis 40 Tage, bevorzugt 4 bis 10 Tage, auf einem festen oder flüssigen Nährboden, beispielsweise Hefe-Malz-Agar oder Kartoffel-Dextrose-Agar, wachsen läßt.

Der Fermentationsverlauf und die Bildung des Cillianons kann entsprechend dem Fachmann bekannten Methoden, wie z. B. durch Austestung der biologischen Aktivität in Bioassays oder durch chromatographische Methoden wie Dünnschichtchromatographie (DC) oder Hochleistungflüssigkeitschromatographie (HPLC) verfolgt werden.

Das Cillianon kann sowohl im Mycel als auch im Kulturfiltrat vorkommen, gewöhnlich befindet sich die Hauptmenge in der Zellmasse (Mycel). Es ist deshalb zweckmäßig, diese durch Filtration oder Zentrifugation vom Filtrat zu trennen. Das Filtrat wird mit einem mit Wasser nicht mischbaren Lösungsmittel wie z.B. 1-Butanol, Essigsäureethylester, Chloroform oder ähnlichem extrahiert. Das Mycel wird zweckmäßigerweise mit Methanol oder Acetonitril extrahiert, es können aber auch die oben genannten mit Wasser nicht mischbaren Lösungsmittel verwendet werden.

Die Extraktionen können in einem weiten pH-Bereich durchgeführt werden, es ist jedoch zweckmäßig, im sauren Milieu, vorzugsweise zwischen pH 0.5 und pH 5 zu arbeiten. Die organischen Extrakte können z.B. im Vakuum konzentriert und getrocknet werden.

Eine Methode der Isolierung des Cillianons ist die Lösungs-Verteilung in an sich bekannter Weise.

Eine andere Methode der Reinigung ist die Chromatographie an Adsorptionsharzen wie z.B. an Diaion® HP-20 (Mitsubishi Casei Corp., Tokyo), an Amberlite® XAD 7 (Rohm and Haas, USA), an Amberchrom® CG, (Toso Haas, Philadelphia, USA) oder an ähnlichen. Geeignet sind darüber hinaus zahlreiche Reverse Phase-Träger, z.B. RP₁₈, wie sie z.B. im Rahmen der Hochdruckflüssigkeits-Chromatographie (HPLC) allgemein bekannt geworden sind.

Eine weitere Reinigungsmöglichkeit für das Cillianon besteht in der Verwendung von sog. straight Phase-Chromatographie-Trägern wie z.B. Kieselgel oder Al₂O₃ oder anderen in an sich bekannter Weise. Geeignet sind hierfür viele Lösungen und ihre Mischungen, wie z.B. Petrolether / Chloroform / Eisessig-Gemische.

Ein alternatives Isolierungsverfahren ist die Verwendung von Molekularsieben, wie z.B. Fractogel® TSK MW-40, Sephadex® LH-20 und andere, in an sich bekannter Weise. Es ist darüber hinaus auch möglich, aus angereichertem Material das Cillianon durch Kristallisation zu gewinnen. Geeignet hierzu sind z.B. organische Lösungsmittel und ihre Gemische, wasserfrei oder mit Wasserzusatz. Ebenso können Zusätze von Säuren, wie z.B. Trifluoressigsäure, HCl, H₂SO₄, Ameisensäure oder andere von Vorteil sein.

Cillianon oder davon abgeleitete chemische Derivate können nach dem Fachmann bekannten Methoden in die entsprechenden pharmakologisch verträglichen Salze übergeführt werden.

Unter pharmakologisch verträglichen Salze der erfindungsgemäßen Verbindungen versteht man sowohl anorganische als auch organische Salze, wie sie in Remington's Pharmaceutical Sciences (17. Auflage, Seite 1418 (1985)) beschrieben sind. Als Salze kommen insbesondere Alkali- und Erdalkalisalze, Salze mit physiologisch verträglichen Aminen und Salze mit anorganischen oder organischen Säuren wie z. B. HCl, HBr, H₂SO₄, Maleinsäure, Fumarsäure in Frage.

Das von Penicillium spec. HAG 259 produzierte, dann teilisolierte oder isolierte Cillianon kann für chemische Modifikationen eingesetzt werden. Modifikationen erfolgen gemäß dem in der Fachliteratur beschriebenen und dem Fachmann bekannten Verfahren. Diese Verfahren sind z. B.
- Hydrierung von Doppelbindungen zwischen Kohlenstoffatomen, aber auch zwischen Kohlenstoff- und Sauerstoffatomen, beispielsweise durch katalytische Hydrierung;
- Veresterungen von Alkohol- und Säuregruppen, bevorzugt zur Herstellung von Alkylestern mit einer Kettenlänge von 1 bis 5, besonders bevorzugt zur Herstellung von Dimethylestern;
- Alkylierung von Kohlenstoff-, Stickstoff- oder Sauerstoffatomen, bevorzugt zur Bildung von Alkylgruppen mit einer Kettenlänge von 1 bis 5 wobei die Alkylgruppen gegebenenfalls substituiert sein können;
- Substitution von Alkoholgruppen durch Alkylmercapto- oder Alkylaminogruppen;
- Acylierung von Alkohol- oder Aminogruppen zu Säureestern bzw. Säureamiden, bevorzugt mit Acylverbindungen einer Kettenlänge von 1 bis 5.

Verbindungen der Formel I, worin R¹ OH und R² -CH₂-CO-CH₃ bedeutet können durch Umsetzung von Cillianon (R¹ und R² sind OH) mit Aceton nach dem Fachmann bekannten Methoden hergestellt werden. Verbindungen der Formel I, worin R¹ OH und R² -NH-CH₂-COOH bedeutet, können bespielsweise durch Umsetzung von Cillianon mit Glycin nach dem Fachmann bekannten Verfahren hergestellt werden.

Die physikalisch-chemisch sowie spektroskopischen Eigenschaften von Cillianon lassen sich wie folgt zusammenfassen:

**Tabelle 1**

| | |
|---|---|
| Aussehen: | dunkelgelbe, in Methanol, Acetonitril, Chloroform und Benzol lösliche Substanz. Im sauren Milieu stabil, jedoch unbeständig in wässrigem Alkali und unter Lichteinwirkung. |
| Summenformel: | C₁₉H₁₈O₇ |
| Molekulargewicht: | 358 |
| ESI-MS: | 359 (M+H)⁺ |
| FAB-MS, HR: | gefunden: 357,0974, berechnet für (M-H₂+H)⁺ 357,0974. |
| | gefunden 341.1021, berechnet für (M-H₂O+H)⁺ 341.1025. |
| UV-Maxima: | 220, 259,5 und 335 nm, gemessen in Acetonitril -Wasser (99:1) mit 0,2 % Trifluoressigsäure (s. Abb. 1), |
| Infrarot-Spektrum: | FT-IR-Spektrum, gemessen als KBr-Pressling (Abb. 2), |
| ¹³C-NMR-Spektrum: | 14.5, 20.6/24.7, 25.6, 43.2, 86.3, 92.5, 108.1, 109.7, 110.8, 118.6, 119.0, 138.6, 151.6, 167.9, 168.0, 168.6, 193.2, 195.5 ppm, gemessen in Deuterochloroform bei 125 MHz, Raumtemperatur. |
| ¹H-NMR-Spektrum: | 1.31, 1.50, 1.54, 2.77, 4.72, 6.74, 13.0 und 13.9 ppm, gemessen in Deuterochloroform bei 500 MHz, Raumtemperatur. |

Es wurde gefunden, daß die erfindungsgemäßen Verbindungen die Bindung von Interleukin-4 (IL-4) an den IL-4-Rezeptor inhibieren und die IL-4-Wirkung unterdrücken. Die erfindungsgemäßen Verbindungen sind somit als Hemmstoffe der IL-4-Wirkung wirksam.

Die IL-4 inhibierende Wirkung der erfindungsgemäßen Verbindungen kann in zellfreien Bindungsassays bestimmt werden.

Aus der deutschen Patentanmeldung DE 43 22 330 A1 ist bekannt, daß durch die Unterdrückung der IL-4 Wirkung Krankheiten diagnostiziert, therapiert und/oder behandelt werden können, die durch ein vermehrtes Auftreten von T-Helferzellen vom TH2-Typ gekennzeichnet sind.

Die Therapie und Prophylaxe vieler allergischer, viraler, parasitärer und bakterieller Erkrankungen stellt nach wie vor ein großes Problem dar. Bei einigen parasitären, viralen und bakteriellen Erkrankungen ist bekannt, daß in ihrem Verlauf Veränderungen in Subpopulationen von lymphozytären und monozytären Zellen auftreten. Dies betrifft zum Beispiel das vermehrte Auftreten von sogenannten T-Helfer-Zellen des Typs 2 (im folgenden TH2-Zellen genannt).
T-Zellen können generell aufgrund von Oberflächenmarkern und aufgrund ihrer Funktion in Subpopulationen unterteilt werden. So tragen beispielsweise T-Helfer-Lymphozyten CD4-Oberflächenmoleküle und sezernieren nach ihrer Aktivierung Cytokine.

Analysen des Cytokin-Musters von klonierten T-Helferzellen aus gesunden Mäusen oder mit allogenen Zellen stimulierten Mäusen ergaben, daß diese Zellen Interleukin-2, Interleukin-4, Gamma-Interferon, Interleukin-5, Interleukin-6, Interleukin-10 und Lymphotoxin produzieren (T-Helfer Zellen vom sogenannten THO-Typ).

Nach Infektion von Mäusen, zum Beispiel mit dem bakteriellen Antigen Brucella abortus oder mit Mycobacterium tuberkulosis, wurden nach Klonierung von T-Helfer-Zellen vor allem Klone gefunden, die Lymphotoxin, Gamma-Interferon und Interleukin-2, aber wenig oder kein Interleukin-4, Interleukin-5, Interleukin-6 und Interleukin-10 sezernieren (T-Helfer-Zellen vom sogenannten TH1-Typ).

Nach Infektion von beispielsweise suszeptiblen Mäusen mit parasitären Erregern wie Leishmania major traten bei Klonierungen von T-Helferzellen vor allem Klone auf, die vermehrt Interleukin-4, Interleukin-5 und Interleukin-10 produzieren, aber verringerte oder nicht detektierbare Mengen von Interleukin-2 und Gamma-Interferon (T-Helferzellen vom TH2-Typ) (Mosmann et al. Immunological Review 1991, No. 123, 209-229; S. Romagnani, Immunology Today, 256-257; Vol. 12, No. 8 1991).

Dieses vermehrte Auftreten von TH-2-Lymphozyten wurde bereits bei einigen Infektionserkrankungen im Tier und im Menschen nachgewiesen (Else and Grenic, Parasitology Today, Vol. 7, No. 11, 1991, pp. 313-316; Parasitology Today, Vol. 7, No. 10, 1991, p. 261) und spiegelt sich auch in sekundären Parameter wieder. So zeigten mit Leishmania major infizierte Mäuse im allgemeinen eine reduzierte Produktion von Gamma-Interferon, stark erhöhtes Serum IgE und Eosinophilie.

Beim Menschen wurde z.B. bei lepromatöser Lepra, Leishmaniasis und Schistosomiasis und Infektionen mit Mycobacterium tuberculosis im allgemeinen stark erhöhte IgE-Konzentration im Serum dieser Patienten im Vergleich zu Seren von Normalpersonen gefunden. Bei den parasitären Infektionen wird oft eine Eosinophilie im Verlauf der Erkrankung beobachtet.

Auch IgE-vermittelte allergische Reaktionen vom Sofort-Typ wie Atopische Dermatitis und Asthma sind durch eine solche Dysregulation gekennzeichnet. Beispielsweise sind antigen-spezifische T-Zell-Klone aus Hautbiopsien von Patienten mit Atopischer Dermatitis vor allem vom TH2-Typ (Kapsenberg et al. Immunology Today, Vol. 12, No. 11, 1991, 392-395).

Die erfindungsgemäßen Verbindungen eignen sich sowohl zur Therapie und Prophylaxe als auch zur Diagnose von Allergien (z.B. als Antiasthmatika, Immunsuppressiva) und Infektionen, insbesondere virale, bakterielle und parasitäre Infektionen sowie Pilzinfektionen; vorzugsweise Infektionen mit dem Humanen Immun-defizienzvirus (HIV), Mycobakterien vor allem Mycobakterium leprae, mit Listerien, mit Protozoen, vor allem der Gattungen Leishmania und Plasmodium, mit Helminthen, vor allem der Gattung Schistosoma, Nipponstrongylus und Heligmosomoides mit Trichurida, Trichinella, Taenia (Cysticercus), Candida und Aspergillus. Es können aber auch allergische Reaktionen vom Sofort-Typ, insbesondere IgE-vermittelte Reaktionen diagnostiziert, behandelt oder prophylaktisch behandelt werden. Vor allem zählen dazu Atopische Dermatitis und Asthma. Ferner sind die erfindungsgemäßen Verbindungen als Antiphlogistika oder auch zum Einsatz im Pflanzenschutz, insbesondere auch als antifungisches Mittel, geeignet.

Die Applikationsformen sind im allgemeinen bei unterschiedlichen Erkrankungen verschieden. So kann beispielsweise die topische Applikation bei einigen Erkrankungen von Vorteil sein. Zum Beispiel ist bei Asthma eine Inhalationsapplikation, bei Conjunctivitis eine Applikation in Augentropfen, bei Atopischer Dermatitis eine dermale oder intradermale Applikation vorteilhaft, da die pathologischen TH2-Zellen vor allem topisch nachgewiesen werden können.

Die erfindungsgemäßen Verbindungen lassen sich auch allgemein als wissenschaftliche Werkzeuge (Tools) einsetzen, um die Bindung von Interleukin 4 (IL- 4) an IL-4-Rezeptoren zu hemmen.

Die Erfindung betrifft weiterhin pharmazeutische Zubereitungen, die eine oder mehrere der erfindungsgemäßen Verbindungen enthalten.
Die Arzneimittel können z.B. in Form von pharmazeutischen Präparaten, die man oral, z.B. in Form von Tabletten, Dragees, Hart- oder Weichgelatinekapseln, Lösungen, Emulsionen oder Suspensionen verabreichen kann, verwendet werden. Der Einschluß der Arzneimittel in Liposomen, die gegebenenfalls weitere Komponenten wie Proteine enthalten, ist eine ebenfalls geeignete Applikationsform. Sie können auch rektal z.B. in Form von Suppositorien oder parenteral z.B. in Form von Injektionslösungen verabreicht werden. Für die Herstellung von pharmazeutischen Präparaten können diese Verbindungen in therapeutisch inerten organischen und anorganischen Trägern verarbeitet werden. Beispiele von solchen Trägern für Tabletten, Dragees und Hartgelatinekapseln sind Laktose, Maisstärke oder Derivate davon, Talk und Stearinsäure oder Salze davon. Geeignete Träger für die Herstellung von Lösungen sind Wasser, Polyole, Saccharose, Invertzucker und Glucose. Geeignete Träger für Injektionslösungen sind Wasser, Alkohole, Polyole, Glycerol und pflanzliche Öle. Geeignete Träger für Suppositorien sind pflanzliche und gehärtete Öle, Wachse, Fette und halbflüssige Polyole. Die pharmazeutischen Präparate können auch Konservierungsmittel, Lösemittel, Stabilisierungsmittel, Netzmittel, Emulgatoren, Süßstoffe, Farbstoffe, Geschmacksmittel, Salze zur Veränderung des osmotischen Drucks, Puffer, Überzugsmittel, Antioxidantien, sowie ggf. andere therapeutische Wirkstoffe enthalten.

Die Erfindung betrifft auch ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Bevorzugte Verabreichungsformen sind orale und topische Applikationen, lokale Applikationen wie beispielsweise mit Hilfe eines Katheters oder aber auch Injektionen.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung oder daraus abgeleiteter chemischer Derivate enthält. Bei festen Dosierungseinheiten wie Tabletten, Kapseln und Suppositorien kann diese Dosis bis zu etwa 200 mg, bevorzugt jedoch etwa 0,1 bis 100 mg, und bei Injektionslösungen in Ampullenform bis zu etwa 200 mg, vorzugsweise aber etwa 0,5 bis 100 mg, pro Tag betragen.

Die zu verabreichende Tagesdosis ist abhängig vom Körpergewicht, Alter, Geschlecht und Zustand des Säugers. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

Die folgenden Beispiele sollen der näheren Erläuterung der Erfindung dienen, ohne die Breite der Erfindung in irgendeiner Weise begrenzen zu wollen. Die folgenden Abkürzungen wurden verwendet:

### Abkürzungen:

- BSA: Rinderserumalbumin
- DMSO: Dimethylsulfoxid
- ELISA: Enzyme-linked Immuno Sorbent Assay
- huIL: human Interleukin
- IL: Interleukin
- PBS: Phosphatpuffersaline
- PBSA: Rinderserumalbumin in PBS

Die Abbildungen sind wie folgt beschriftet:

### Abbildung 1:

UV-Spektrum in Acetonitril-H₂O (99:1), 0,2 % Trifluoressigsäure

### Abbildung 2:

IR-Spektrum (FT-IR), gemessen als KBr-Pressling

### Abbildung 3:

IL-4 Rezeptortest ■ Cillianon

### Beispiele:

### Beispiel 1:

### Herstellung einer Sporensuspension des Produzentenstammes

100 ml Nährlösung (20 g Malzextrakt, 2 g Hefeextrakt, 10 g Glucose, 0,5 g (NH₄)₂HPO₄ in 1 l Leitungswasser, pH-Wert vor der Sterilisation 6,0) in einem 500 ml sterilen Erlenmeyerkolben werden mit dem Stamm beimpft und 72 Stunden bei 25°C und 140 UpM auf einer rotierenden Schüttelmaschine inkubiert. Anschließend werden 20 ml Kulturflüssigkeit in einem sterilen 500 ml Erlenmeyerkolben mit dem Nährboden (Kartoffelinfus 4,0 g/l [Infus aus 200 g Kartoffeln in 1000 ml H₂O] 20,0 g/l D-Glucose, pH vor der Sterilisation 5,6) dem zusätzlich 15 g Agar/l zur Verfestigung zugegeben wurde, gleichmäßig verteilt und dekantiert. Die Kulturen werden 10 bis 14 Tage bei 25°C inkubiert. Die nach dieser Zeit entstandenen Sporen eines Kolbens werden mit 500 ml entionisiertem Wasser, das einen Tropf eines handelsüblichen nichtionischen Tensids (z.B. ®Triton X 100, Fa. Serva) enthält, abgeschwemmt, sofort weiterverwendet oder bei -22°C in 50 % Glycerin aufbewahrt.

### Beispiel 2:

### Herstellung einer Kultur bzw. einer Vorkultur des Produzentenstammes im Erlenmeyerkolben

Ein steriler 500 ml Erlenmeyerkolben mit 100 ml der unter a) beschriebenen Nährlösung wird mit einer auf einem Schrägröhrchen gewachsenen Kultur oder mit 0,2 ml Sporensuspension angeimpft und auf einer Schüttelmaschine im Dunkeln bei 140 UpM und 25°C inkubiert. Die maximale Produktion der Verbindungen der Formel I ist nach ca.

96 Stunden erreicht. Zum Animpfen von 10 und 100 l Fermentern genügt eine 72 Stunden alte Submerskultur (Animpfmenge ca. 5 %) aus der gleichen Nährlösung.

### Beispiel 3

### Herstellung von Cillianon

Ein 10 l Fermenter wird unter folgenden Bedingungen betrieben:

| | |
|---|---|
| Nährmedium: | 0,5 g/l Caseinpepton |
| | 0,5 g/l Fleischpepton |
| | 1,0 g/l Glucose |
| | 1,8 g/l Maltose |
| | pH 6,0 (vor der Sterilisation) |
| Inkubationszeit: | 96 Stunden |
| Inkubationstemperatur: | 25°C |
| Rührergeschwindigkeit: | 200 UpM, unter Lichtausschluß |
| Belüftung: | 5 l Luft/min. |

Durch wiederholte Zugabe weniger Tropfen ethanolischer Polyollösung kann die Schaumbildung unterdrückt werden. Das Produktionsmaximum wird nach ca. 96 Stunden erreicht.

### Beispiel 4:

### Isolierung des Cillianon

9 l der nach Beispiel 2 gewonnnen Kulturlösung wurden abzentrifugiert und die Zellmasse (∼ 1,5 l) zweimal mit je 3 l Acetonitril, dem 0,5 % bzw. 0,1 % Trifluoressigsäure hinzugefügt worden ist, ausgerührt. Die vereinigten Extrakte wurden im Vakuum konzentriert, in n-Butanol aufgenommen, abzentrifugiert und der klare, wirkstoffhaltige Überstand erneut unter vermindertem Druck vom Lösungsmittel befreit. Der Rückstand wurde mit reinem Hexan digeriert, 20 Stunden bei + 1°C stehen gelassen und der entstandene wirkstoffhaltige Niederschlag gesammelt (2.3 g).

### Beispiel 5

### Reinigung des Cillianon

2 g des nach Beispiel 3 gewonnenen Produktes wurden in 30 %igem Acetonitril/Wasser mit 1 % Trifluoressigsäure gelöst und auf eine 150 ml fassende, mit MCI Gel CHP20P (75 bis 150 µ, Mitsubishi Kasei Corp. Tokyo) gefüllte Säule aufgetragen. Eluiert wurde mit einem Gradienten von 30 % bis 60 % Acetonitril in 0.05 M Trifluoressigsäure. Die Cillianon-haltigen Fraktionen wurden zusammengefaßt, nach Konzentrieren im Vakuum auf 250/32 Nucleosil® 100-7 C₁₈ AB (Macherey-Nagel, Düren) aufgereinigt. Als Elutionsmittel diente 20 % bis 60 % Acetonitril in 1 % Trifluoressigsäure. Die reinen Cillianon enthaltenden Fraktionen wurden gefriergetrocknet (60 mg, Reinheit 98 %).

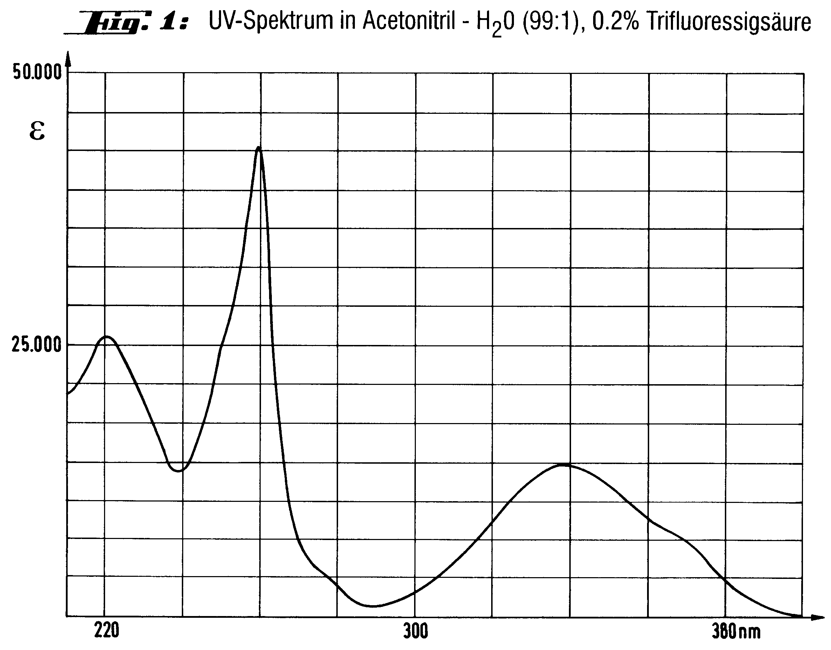

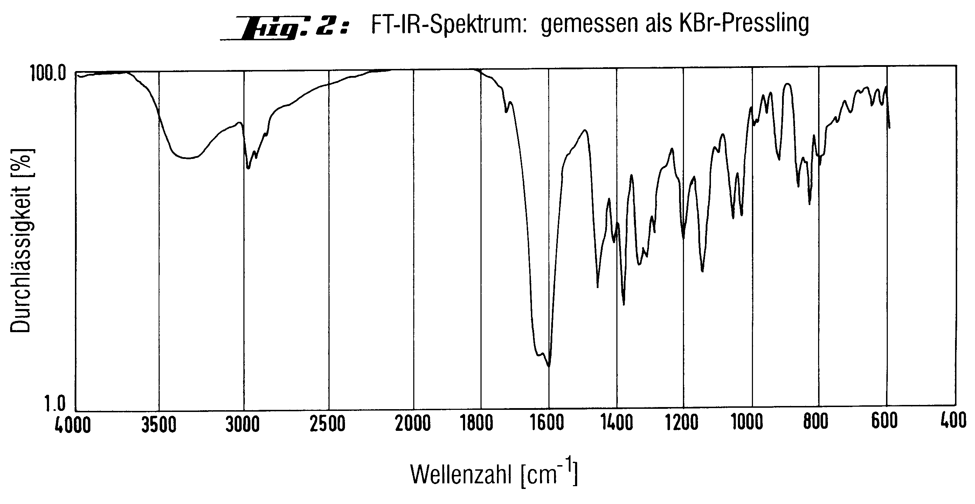

Daten der Massenspektrometrie:
ESI-MS: 359 (M+H)⁺, entsprechend der Summenformel C₁₉H₁₈O₇
FAB-MS, HR: gefunden: 357,0974, berechnet für (M-H₂+H)⁺ 357,0974. und gefunden 341.1021, berechnet für (M-H₂O+H)⁺ 341.1025.
NMR-Signale:
¹³C-NMR-Signale sind bei:
14.5, 20.6/24.7, 25.6, 43.2, 86.3, 92.5, 108.1, 109.7, 110.8, 118.6, 119.0, 138.6, 151.6, 167.9, 168.0, 168.6, 193.2, 195.5 ppm, gemessen in Deuterochloroform bei 125 MHz.
¹H-NMR-Signale sind bei:
1.31, 1.50, 1.54, 2.77, 4.72, 6.74, 13.0 und 13.9 ppm, gemessen in Deuterochloroform bei 500 MHz.
- UV:: siehe Abb. 1
- IR:: siehe Abb. 2

### Beispiel 6:

### Spezifische Inhibition der IL-4 Bindung in zellfreien Bindungsassays

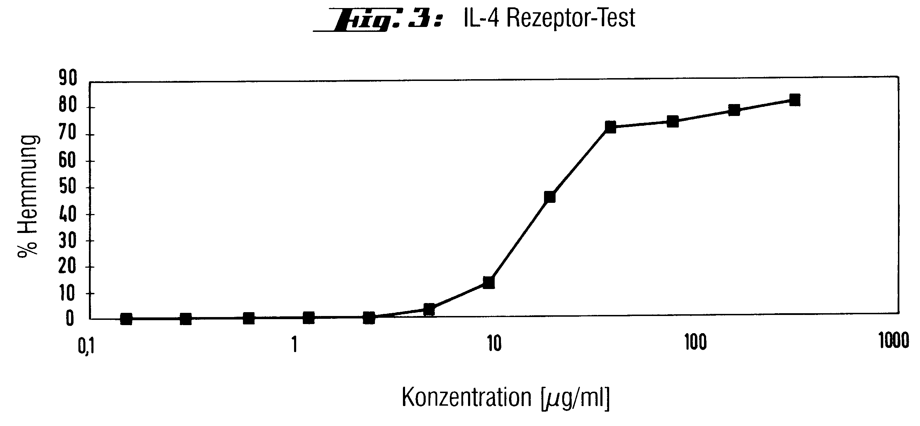

Aus der EP 488 170 A1 sind zellfreie Bindungsteste bekannt. Für ihre Durchführung werden rekombinante chimäre Proteine verwendet, die aus der extrazellulären Region von normalerweise membranständigen Rezeptoren bestehen, an deren Carboxyterminus die Fc-Region einer schweren Immunglobulinkette bestehend aus Hinge-, CH2- und CH3-Domäne fusioniert wurde. Diese sogenannten Rezeptor/Fc-Fusionsproteine können unter Erhalt der spezifischen Rezeptorbindungsaktivität an Festphasen gebunden werden, die beispielsweise mit monoklonalen Antikörpern gerichtet gegen den Fc-Teil vorbeschichtet wurden. Als Festphase wurden für dieses Beispiel ®Nunc Typ B ELISA-Platten verwendet. Rezeptor/Fc-Fusionsproteine gelöst in PBS enthaltend 10 mg/ml BSA (PBSA) wurden an diese vorbehandelten Platten gebunden (huIL-4R/Fc: 500 ng/ml; muIL-4R/Fc: 250 ng/ml; huIL-1R/Fc: 125 ng/ml; 1 h bei Raumtemperatur). Nach Waschen wurden die Peptide beziehungsweise die jeweiligen spezifischen unmarkierten Liganden (huIL-4R/Fc: huIL-4; muIL-4R/Fc: muIL-4; huIL-1R/Fc:huIL-1ra) in variierenden Konzentrationen und danach die spezifischen Liganden in biotinylierender Form in einer festen Konzentration zugegeben (100 ng/ml). Die Inkubation erfolgte 1 h bei Raumtemperatur in PBSA enthaltend 5 % DMSO. Nach Waschen erfolgte eine Inkubation mit Streptavidin/Peroxidase (Amersham, 1:2000 in PBSA) für 20 min bei Raumtemperatur. Der Nachweis der gebundenen Peroxidase geschah nach abermaligen Waschen in einer Tetramethylbenzidin-Substratlösung (Behringwerke). Nach 30 min Inkubation wurde die Extinktion bei 450 nm gemessen, die direkt die Menge des an den Rezeptor gebundenen biotinylierter Liganden reflektiert. Für die Abbildung 3 wurde das gemessene Extinktionssignal in Abhängigkeit von der Konzentration des kompetierenden Agens aufgetragen, wobei als 100 %-Wert die gemessene Extinktion in Abwesenheit eines kompetierenden Agens herangezogen wurde.

Abb. 3 zeigt, daß im huIL-4 Bindungstest unmarkiertes huIL-4 mit biotinyliertem huIL-4 um die Bindung an den humanen IL-4 Rezeptor kompetiert.

Der IC₅₀ = 20 µg/mL entspricht 55,9 µmol/mL

## Patentansprüche

1. Verbindung der Formel I 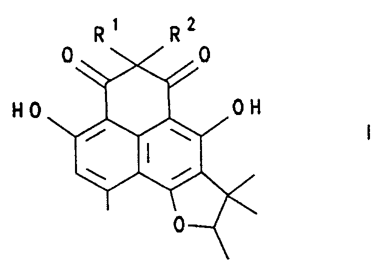 in welcher bedeuten:
a)
R¹ und R² unabhängig voneinander
-CₙH₂ₙ-R³, -O-R³, -S-R³, oder NH-R³;
R³ Wasserstoff, (C₁-C₆)-Alkanoyl oder einen substituierten oder unsubstituierten (C₁-C₆₎-Alkylrest; und
n 1,2,3,4,5 oder 6; oder
b) R¹ und R² zusammen mit dem sie tragenden C-Atom einen substituierten oder unsubstituierten alicyclischen Rest mit 3 - 9 C-Atomen bilden,
sowie deren physiologisch verträglichen Salze.

2. Verbindung der Formel I gemäß Anspruch 1, worin
R¹ OH;
R² OH, NH-R³ oder CH₂-R³; und
R³ Wasserstoff, Formyl, Acetyl, Carboxymethyl oder Carboxyethyl bedeuten, sowie deren pharmakologisch verträglichen Salze.

3. Verbindung der Formel I gemäß einem oder mehreren der Ansprüche 1-2, worin R¹ und R² Hydroxyl bedeutet, sowie deren pharmakologisch verträglichen Salze.

4. Verbindung der Summenformel C₁₉H₁₈O₇ (Cillianon), erhältlich aus Penicillium spec. HAG 0259 durch Fermentierung von Penicillium spec. HAG 0259 in einem Kulturmedium, bis sich Cillianon in der Kulturbrühe anhäuft, und anschließender Isolierung und Aufreinigung des Wirkstoffes, sowie dessen pharmakologisch verträglichen Salze.

5. Chemische Derivate, abgeleitet aus einer Verbindung der Summenformel C₁₉H₁₈O₇ (Cillianon), erhältlich aus Penicillium spec. HAG 0259 durch Fermentierung von Penicillium spec. HAG 0259 in einem Kulturmedium, bis sich Cillianon in der Kulturbrühe anhäuft, anschließender Isolierung und Aufreinigung des Wirkstoffes und Überführung in chemische Derivate, sowie deren pharmakologisch verträglichen Salze.

6. Verfahren zur Herstellung einer Verbindung gemäß einem oder mehreren der Ansprüche 1-5, dadurch gekennzeichnet, daß man Penicillium spec. HAG 0259 oder dessen Mutanten und/oder Varianten in einem Kulturmedium fermentiert, bis sich das Cillianon in der Kulturbrühe anhäuft, dieses aus der Kulturbrühe isoliert und gegebenenfalls in chemische Derivate überführt.

7. Verfahren gemäß Anspruch 6, dadurch gekennzeichnet, daß man Penicillium spec. HAG 0259 oder dessen Mutanten und/oder Varianten in einem eine Kohlenstoff- und Stickstoffquelle sowie die üblichen anorganische Salze und Spurenelemente enthaltenden Kulturmedium unter aeroben Bedingungen fermentiert.

8. Verfahren gemäß einem oder mehreren der Ansprüch 6-7, dadurch gekennzeichnet, daß die Fermentierung für 24 - 300 Stunden bei 15 - 37EC in einem Nährmedium, das 0,1 - 3 % Caseinpepton, 0,1 - 3 % Fleischpepton, 0,1 - 5 % Glukose und 0,1 - 7 % Maltose enthält und einen pH-Wert zwischen 1 und 7 aufweist, erfolgt.

9. Verfahren gemäß einem oder mehreren der Ansprüche 6-8, dadurch gekennzeichnet, daß das Nährmedium 0,2 - 1 % Caseinpepton, 0,2 - 1 % Fleischpepton, 0,5 - 2 % Glukose und 1 - 2 % Maltose enthält und man die Kultivierung bei einem pH-Wert zwischen 1 und 7 und einer Temperatur von 15 -30EC über 24 - 300 Stunden durchführt.

10. Verfahren gemäß einem oder mehreren der Ansprüche 6-9, dadurch gekennzeichnet, daß man die Kultivierung bei einem pH-Wert zwischen 2,5 und 4,5 und einer Temperatur zwischen 20 und 30EC über 36 - 140 Stunden durchführt.

11. Arzneimittel, enthaltend mindestens eine Verbindung gemäß einem oder mehreren der Ansprüche 1-5 und einen oder mehrere physiologisch annehmbare Träger sowie gegebenfalls geeignete Zusatzstoffe und/oder Hilfsstoffe.

12. Verfahren zur Herstellung eines Arzneimittels gemäß Anspruch 11, dadurch gekennzeichnet, daß mindestens eine wirksame Verbindung gemäß einem oder mehreren der Ansprüche 1-5 mit einem physiologisch annehmbaren Träger sowie gegebenenfalls geeigneten Zusatzstoffen und/oder Hilfsstoffen vermischt wird.

13. Verwendung einer Verbindung gemäß einem oder mehreren der Ansprüche 1 bis 5 zur Herstellung eines Arzneimittels zur Behandlung und Prophylaxe von allergischen, viralen, parasitären und bakteriellen Erkrankungen sowie von Pilzinfektionen.

14. Verwendung einer Verbindung gemäß einem oder mehreren Ansprüchen der Ansprüche 1 bis 5 als pharmakologisch wirksamen Stoff oder als wissenschaftliches Wekzeug (Tool) zur Hemmung der Bindung von Interleukin-4 (IL-4) an den IL-4-Rezeptor.

15. Penicillium spec. HAG 0259 (DSM 9791)
